**Europäisches Patentamt**

**European Patent Office**

**Office européen des brevets**

(19)

(11) Veröffentlichungsnummer: **0 135 598
B1**

(12)

# EUROPÄISCHE PATENTSCHRIFT

(45) Veröffentlichungstag der Patentschrift:
07.10.87

(21) Anmeldenummer: **83109499.0**

(22) Anmeldetag: **23.09.83**

(51) Int. Cl.⁴: **A 61 B 3/10,** G 01 B 3/18

(54) Werkzeug zum Messen des Augenabstandes von der Nasenwurzel.

(43) Veröffentlichungstag der Anmeldung:
**03.04.85 Patentblatt 85/14**

(45) Bekanntmachung des Hinweises auf die Patenterteilung:
**07.10.87 Patentblatt 87/41**

(84) Benannte Vertragsstaaten:
**AT BE CH FR GB IT LI LU NL SE**

(56) Entgegenhaltungen:
DE - A - 3 218 489
US - A - 1 588 401
US - A - 1 935 175
US - A - 3 058 225
US - A - 3 495 897
US - A - 3 966 310
US - A - 4 255 861

(73) Patentinhaber: **Pehart, Paul, Kapuzinerstrasse 35,
D-8000 München 5 (DE)**

(72) Erfinder: **Pehart, Paul, Kapuzinerstrasse 35,
D-8000 München 5 (DE)**

(74) Vertreter: **Staeger, Sigurd, Dipl.-Ing. et al,
Patentanwälte Dr.-Ing. H. Fincke Dipl.-Ing. H. Bohr
Dipl.-Ing. S. Staeger Dipl.-Ing. Dipl.-Wirtsch.-Ing. R.
Sperling Müllerstrasse 31, D-8000 München 5 (DE)**

## Beschreibung

Die Erfindung bezieht sich auf ein Werkzeug zum Messen des Augenabstandes von der Nasenwurzel mit einer Nasenauflage.

Bei einem aus der US-A-1 935 175 bekannten Messwerkzeug dieses Typs befindet sich die Nasenauflage als keilförmige, etwa im Mittelbereich zwischen den beiden Enden eines linealähnlichen, im wesentlich die Form eines Flachquaders aufweisenden Gehäuses. Beiderseits der Ausnehmung ist das Gehäuse mit einer Durchtritts-Sichtöffnung versehen. Der Abstand dieser beiderseitigen Durchtritts-Sichtöffnungen von der Gehäuseausnehmung entspricht jeweils etwa dem Augenabstand von der Nasenwurzel eines Menschen. Ebenfalls beiderseits der Gehäuseausnehmung ist jeweils ein Schieber aus einem durchsichtigen Werkstoff innerhalb des Gehäuses verschiebbar gelagert.

Die in der Regel horizontale Verschieberichtung entspricht der Abstandsrichtung zwischen den Durchtritts-Sichtöffnungen und der Gehäuseausnehmung. Im Bereich der Durchtritts-Sichtöffnungen sind die beiden Schieber jeweils mit einer rechtwinklig zur Verschieberichtung, also vertikal verlaufenden Messmarke bzw. einem Fadenkreuz versehen. Ausserhalb der Durchtritts-Sichtöffnungen weisen beide Schieber eine zusätzliche Messmarkierung auf, die mit einer ortsfesten Skaleneinteilung am Gehäuse korrespondiert. Der Abstand des linken oder rechten Auges von der Nasenwurzel eines Menschen wird dadurch gemessen, dass man die Fadenkreuze der beiden Schieber nacheinander auf die Pupillenmitten einstellt und über die Messmarkierungen der beiden Schieber jeweils an den gehäuseseitigen Skalen den Augenabstand misst. Derartige Messwerkzeuge werden in erster Linie zum Anpassen eines Brillengestells an das menschliche Gesicht verwendet.

Der Erfindung liegt die Aufgabe zugrunde, bei einem Werkzeug des eingangs genannten Typs eine von beiden Seiten ablesbare numerische Anzeige so auszubilden, dass sie in einem stärkeren Griffteil untergebracht werden kann.

Diese Aufgabe wird durch die Kennzeichnungsmerkmale des Anspruchs 1 gelöst.

Durch diese Lösung ist es möglich, den Abstand sowohl des linken als auch des rechten Auges von der Nasenwurzel mit demselben Schieber zu messen.

Die eigentliche Messung, d.h. die Einstellung der dem Messergebnis entsprechenden Relativstellung zwischen Schieber und Gehäuse wird stromlos vorgenommen.

Erst zum Ablesen des Messergebnisses wird ein Druckknopf eines Druckknopfschalters in seine Einschaltstellung gedrückt. Ein Nachlassen dieses Druckes bewirkt eine sofortige Stromabschaltung, d.h. ein Erlöschen der beidseitigen numerischen Messanzeigen.

Auf der Zeichnung sind beispielsweise Ausführungsformen des Erfindungsgegenstandes dargestellt; sie werden nachfolgend näher erläutert; es zeigt:

Fig. 1 eine Seitenansicht des Messwerkzeuges,

Fig. 2 eine Unteransicht des Messwerkzeuges entsprechend Pfeil II in Fig. 1,

Fig. 3 eine Draufsicht auf das Messwerkzeug entsprechend Pfeilrichtung III in Fig. 1,

Fig. 4 eine Draufsicht auf das Vorderteil einer geöffneten Kunststoffhalbschale des Gehäuses des Messwerkzeuges mit einer Darstellung des Verschiebeantriebes entsprechend Pfeil IV in Fig. 2,

Fig. 5 eine perspektivische Ansicht einer anderen Ausführungsform des Messwerkzeuges,

Fig. 6 eine Seitenansicht,

Fig. 7 einen Vertikalschnitt,

Fig. 8 eine Stirnansicht entsprechend Pfeil VIII in Fig. 6 der Schnecke des Spindelantriebes,

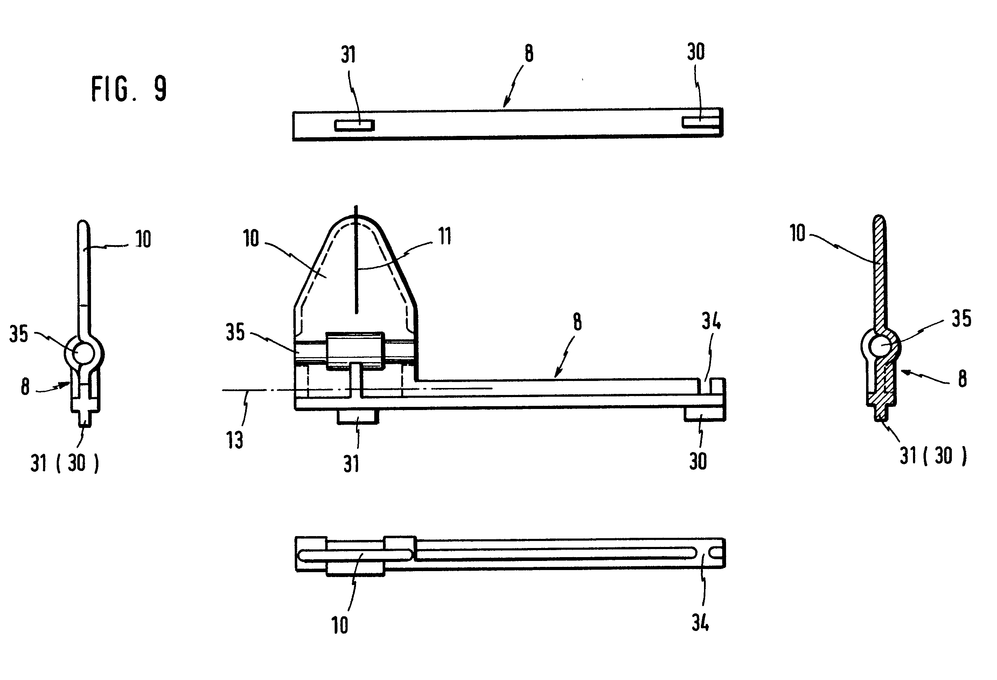

Fig. 9 in sämtlichen Ansichten eine Darstellung des Schiebers,

Fig. 10 eine Seitenansicht einer weiteren Ausführungsform mit auswechselbarer Nasenauflage und

Fig. 11 eine Draufsicht entsprechend Pfeil XI auf die Ausführungsform gemäss Fig. 10.

Das Messwerkzeug besteht im wesentlichen aus dem Gehäuse 1, welches seinerseits das Griffteil 2 und das Verstellteil bzw. Messteil 3 enthält. Das Gehäuse 1 ist mit einer Nasenauflage bzw. etwa winkelförmigen Ausnehmung 4 zum Aufsetzen auf die Nasenwurzel versehen. Die Flanken 5, 6 der Ausnehmung 4 sind Teil eines Nasenstegs. Das Gehäuse 1 ist auf einer Seite mit einem Sichtfenster 7 für eine numerische Messanzeige versehen.

Innerhalb des Gehäuses 1 ist ein Schieber 8 in Axialrichtung 9 verschiebbar gelagert. Der Schieber 8 besteht aus durchsichtigem Werkstoff, insbesondere Kunststoff, und trägt eine einstückig angeformte Scheibe 10 mit einem Fadenkreuz bzw. einer Messmarke 11 oder weist einen schmalen Schlitz auf.

Durch Längsverschiebung des Schiebers 8 in Axialrichtung 9 ist der Abstand der Messmarke 11 zur Gehäuseausnehmung 4 verstellbar.

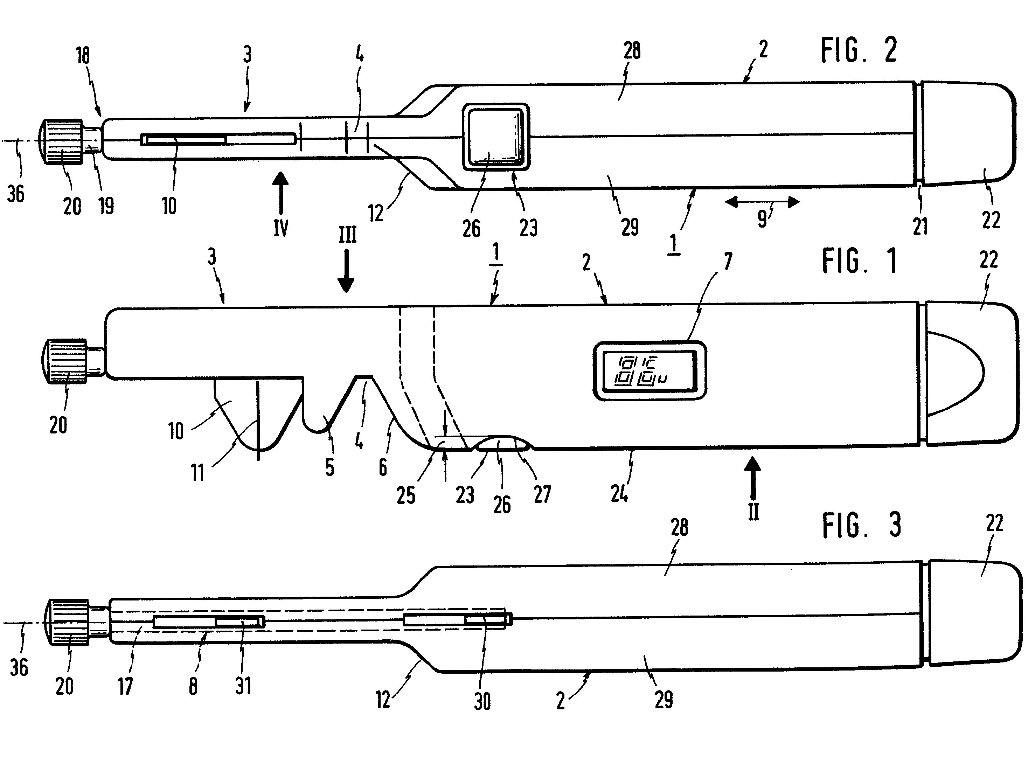

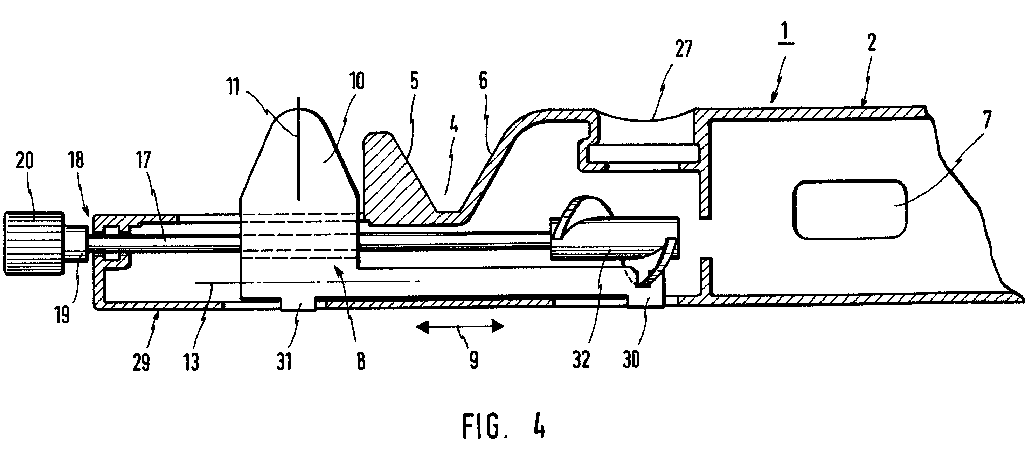

Die Ausnehmung 4 ist an dem der Scheibe 10 zugewandten Ende 12 des den volumenmässig wesentlichen Gehäuseteil bildenden stabähnlichen Griffteiles 2 angeordnet. Die Messscheibe 10 mit der Messmarke 11 ist auf der dem Griffteil 2 abgewandten Seite der Gehäuseausnehmung 4 positioniert. Das Griffteil 2 weist beiderseits der durch die Schieberlängsachse 13 und die Messscheibe 10 bzw. deren Messmarke 11 gebildeten Längsebene jeweils ein Sichtfenster 7 (Fig. 1 und 4) auf, so dass die Messanzeige beim Ausführungsbeispiel von beiden Gehäuseseiten her ablesbar ist; in bestimmten Fällen reicht jedoch auch ein Sichtfenster nur auf einer Seite.

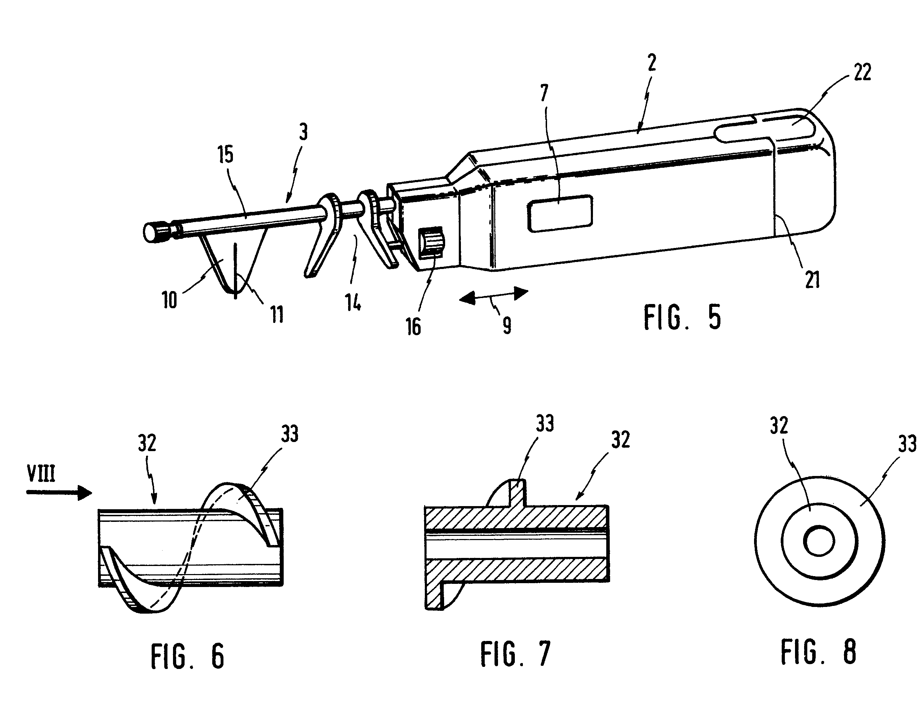

Bei der in Fig. 5 dargestellten Ausführungsform des Messwerkzeuges ist die die Messmarke 11 tragende Messscheibe 10 starr mit dem Griffteil 2 verbunden, während die den Nasensteg bildende Nasenauflage 14 in Axialrichtung 9 durch den innerhalb des Griffteiles 2 angeordneten Schieber (nicht dargestellt) auf dem mit dem Griffteil 2 fest verbundenen Ausleger 15 verschiebbar ist. Der Verschiebeantrieb erfolgt über die Rändelschraube 16, die am der Massscheibe 10 zugewandten Ende des Griffteiles 2 angeordnet ist.

Parallel zur Schieberlängsachse 13 ist eine Stellspindel 17 zur Verstellung des Abstandes zwischen Nasenauflage bzw. Ausnehmung 4 bzw. 14 und Messscheibe 10 im Gehäuse 1 drehbar gelagert. Das Griffende 19 der Stellspindel 17 ist bei der Ausführungsform gemäss Fig. 1–4 am freien Ende 18 des Messteiles 3 angeordnet. Auf das Griffende 19 der Stellspindel 17 ist eine auf ihrem Umfang mit einer Rändelung versehene Griffhülse 20 aufgesetzt.

In den beiden Sichtfenstern 7 ist das Sichtfeld einer numerischen Messanzeige angeordnet. Das Griffteil 2 ist an seinem rückwärtigen freien Ende 21 mit einer durch eine Kappe 2 verschliessbaren Öffnung zur Aufnahme oder Einlage einer elektrischen Batterie zur Erzeugung des Stromes für die Messanzeige versehen. Zum Ein- und Ausschalten des Stromes dient ein Schalter. Der Schalter ist ein in rechtwinklig zur Schiebelängsachse verlaufender Ausschaltrichtung federbelasteter Druckknopfschalter, dessen Druckbetätigungsende 23 aus der Unterseite 24 des Griffteiles 2 vorsteht. Das Vorstehmass 25 entspricht etwa dem Verschiebehub des Druckknopfes 26 zu seiner Überführung aus der Ausschaltstellung (Fig. 1) in die Einschaltstellung.

Die Wandung des Gehäuses 2 ist im Bereich des Druckknopfaustrittes mit einer der Grösse des Vorstehmasses 25 entsprechend tiefen Ausmuldung 27 versehen. Der Druckknopfschalter befindet sich am gehäuseausnehmungsseitigen Ende des Griffteiles 2.

Das Gehäuse 1 besteht aus zwei Kunststoffhalbschalen, deren eine teilweise in Fig. 4 dargestellt ist. Die Teilfugenebene zwischen den beiden Kunststoffhalbschalen verläuft etwa in der Schieberlängsebene, d.h. in Richtung der Mittellängsachse 36 des Messwerkzeuges lotrecht zur Zeichnungsebene in Fig. 2 und 3. Die Messscheibe 10 mit der Messmarke 11 ragt aus dem Gehäuse heraus. Der Schieber 8 ist im wesentlichen plattenförmig ausgebildet und zwischen den beiden Kunststoffhalbschalen 28, 29 in deren Teilfugenebene verschiebbar geführt.

Der Schieber 8 ist mit seitlichen Vorsprüngen 30, 31 einerseits und seinem die Messscheibe 10 bildenden Vorsprung andererseits in Gehäuseschlitzen geführt, die durch entsprechende Aussparungen an den einander gegenüberliegenden Seitenkanten der Kunststoffhalbschalen 28, 29 gebildet sind.

Der Querschnitt des Griffteiles 2 ist etwa rechteckförmig oder elliptisch mit in Richtung der Ellipsenachsen eingedrückten Ellipsenbögen, wobei die längere Rechteckseite bzw. Ellipsenachse in Richtung der Teilfugen- bzw. Mittellängsebene des Messwerkzeuges verläuft.

Auf das griffteilseitige Ende der Stellspindel 17 ist die Schnecke 32 drehfest aufgesetzt. Ihr Schneckengewindegang 33 kämmt mit einer Ausnehmung 34 im Schieber 8. Der Schieber 8 ist im Bereich der Messscheibe 10 von der Stellspindel 17 durchsetzt. Hierzu ist in der Scheibe 10 eine Durchtrittsausnehmung 35 vorgesehen.

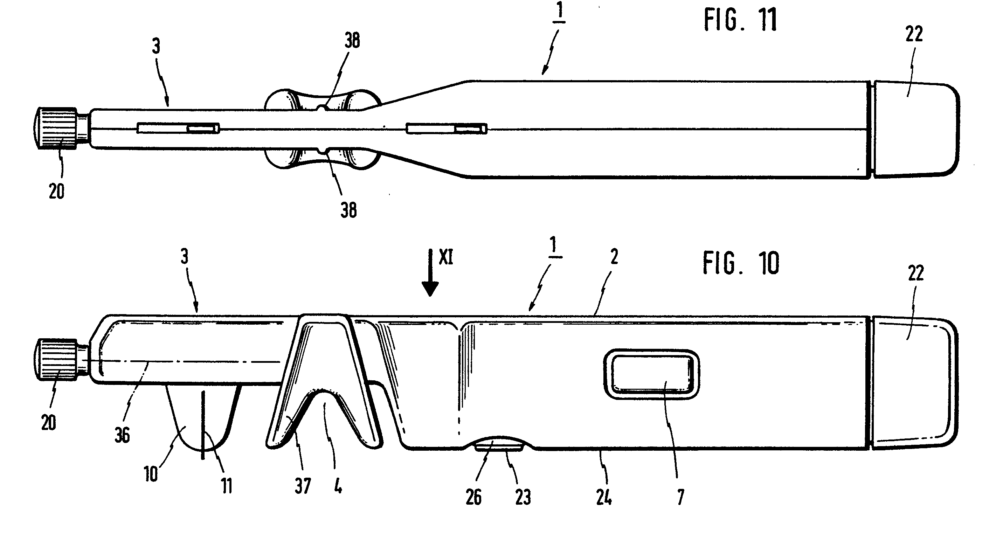

Bei der Ausführungsform gemäss Fig. 10 und 11 ist die Nasenauflage 4 Bestandteil eines am Gehäuse 1 anbringbaren Auswechselteiles 37, welches ortsfest an das Gehäuse 1 anklipsbar ist. Zu diesem Zweck weist das Gehäuse 1 bzw. sein Messteil 3 an seinen beiden Flanken jeweils eine Formschlusserhebung 38 auf, die beispielsweise als rechtwinklig zur Mittellängsachse 36 des Messwerkzeuges verlaufende, schienenartige Erhebung ausgebildet ist. Das Auswechselteil 37 ist dann von der Unterseite 24 des Gehäuses 1 bzw. seines Messteiles 3 her auf das Gehäuse 1 bzw. dessen Messteil 3 vorschlüssig aufschiebbar. Die Ausnehmung 4 des die Nasenauflage bildenden Auswechselteiles 37 ist unterschiedlich entsprechend der Nasenformen ausgebildet. So wird zu ein und demselben Messwerkzeug eine Mehrzahl von unterschiedlichen Nasenformen entsprechenden Auswechselteilen geliefert. Vor der Messung wird jeweils das zur Nase des Messobjektes passende Auswechselteil ausgewählt und an das Gehäuse 1 bzw. sein Messteil 3 angeklipst, bevor der Messvorgang vorgenommen wird.

Zur Messung der Distanz eines Auges von der Nasenwurzel wird das Gehäuse 1 mit der Ausnehmung 4 auf die Nasenwurzel des zu vermessenden Gesichtes aufgesetzt. Sodann wird durch Drehung der Stellspindel 17 an der Griffhülse 20 mit der Messscheibe 10 die Messmarke 11 in Überdeckung mit der Pupille des Auges gebracht. Ist die Lage der Pupille mit der Messmarke 11 genau eingepeilt, so wird das Messgerät vom Gesicht abgenommen. Um den der eingepeilten Pupillendistanz entsprechenden Messwert im Sichtfenster 7 ablesen zu können, wird der Druckknopf 26 gedrückt, durch welchen die Stromversorgung der numerischen Messanzeige eingeschaltet wird. Ist der Messwert des einen Auges festgestellt, wird das Werkzeug in seiner Horizontallage um eine vertikale Achse um etwa 180° geschwenkt und in die andere Hand der Bedienungsperson genommen. Es wird wiederum – nunmehr von der anderen Seite her – die Gehäuseausnehmung 4 auf die Nasenwurzel aufgesetzt und in der vorstehend beschriebenen Weise der zweite Messvorgang für das andere Auge vorgenommen. Die dem Messergebnis entsprechende Messanzeige kann wiederum lediglich nach Druckknopfbetätigung dem Sichtfenster 7 auf der der Bedienungsperson zugewandten Seite des Griffteiles 2 abgelesen werden.

## Patentansprüche

1. Werkzeug zum Messen des Augenabstandes von der Nasenwurzel mit einer Nasenauflage (4, 14), dadurch gekennzeichnet, dass das Werkzeug unsymmetrisch ausgebildet ist und lediglich auf einer Seite der Nasenauflage (4, 14) eine Messmarke (11) aufweist, die mittels eines von einer Stellspindel (17) bewegbaren Schiebers (8) gegenüber der Nasenauflage (4, 14) verstellbar ist, und dass auf der anderen Seite der Nasenauflage (4, 14) ein Griffteil (2) vorgesehen ist, das auf jeder seiner im Gebrauch der Bedienungsperson zugewandten Seiten je ein Sichtfenster (7) für eine numerische Messanzeige aufweist.

2. Werkzeug nach Anspruch 1, dadurch gekennzeichnet, dass das Griffteil (2) mit einer verschliessbaren Öffnung zur Aufnahme mindestens einer elektrischen Batterie versehen ist.

3. Werkzeug nach Anspruch 2, dadurch gekennzeichnet, dass ein Schalter für die numerische Messanzeige ein in Ausschaltrichtung federbelasteter Druckknopfschalter ist.

4. Werkzeug nach einem der Ansprüche 1 bis 3, dadurch gekennzeichnet, dass das Griffteil (2) aus zwei Kunststoffhalbschalen (28, 29) besteht.

5. Werkzeug nach einem der Ansprüche 1 bis 4, dadurch gekennzeichnet, dass eine im Verstellteil (3) angeordnete, durchsichtige Messscheibe (10) neben der Nasenauflage (4, 14) angeordnet ist.

6. Werkzeug nach einem der Ansprüche 2 bis 5, dadurch gekennzeichnet, dass der im Verstellteil (3) gelagerte Schieber (8) plattenförmig ausgebildet ist.

7. Werkzeug nach einem der Ansprüche 1 bis 8, dadurch gekennzeichnet, dass die Nasenauflage (4, 14) Bestandteil eines am Gehäuse (1) oder am Verstellteil (3) anbringbare Auswechselteil (37) ist.

8. Werkzeug nach Anspruch 7, dadurch gekennzeichnet, dass mehrere Auswechselteile (37) mit unterschiedlichen Ausnehmungen (4, 14) für die Nase versehen sind.

**Revendications**

1. Dispositif de mesure de l'écart entre l'œil et la racine du nez avec un appui nasal (4, 14), caractérisé en ce que ce dispositif a une forme dissymétrique et présente d'un seul côté de l'appui nasal (4, 14) un repère de mesure (11) qui est réglable par rapport audit appui nasal (4, 14) au moyen d'un curseur (8) mobile par une broche de réglage (17), et en ce qu'il comporte de l'autre côté de l'appui nasal (4, 14) une partie poignée (2) présentant une fenêtre de lecture (7) d'un affichage de mesure numérique sur chacun des côtés susceptibles d'être tournés vers l'opérateur.

2. Dispositif selon la revendication 1, caractérisé en ce que la partie poignée (2) est pourvue d'une ouverture obturable pouvant recevoir au moins une pile électrique.

3. Dispositif selon la revendication 2, caractérisé en ce qu'un commutateur pour l'afficheur numérique est un interrupteur à bouton-poussoir avec ressort de rappel.

4. Dispositif selon l'une des revendications 1 à 3, caractérisé en ce que la partie poignée (2) est composée de deux demi-coquilles (28, 29) en matière plastique.

5. Dispositif selon l'une des revendications 1 à 4, caractérisé en ce qu'un disque de mesure transparent (10) est disposé dans la partie de réglage (3) à côté de l'appui nasal (4, 14).

6. Dispositif selon l'une des revendications 2 à 5, caractérisé en ce que le curseur (8) disposé dans la partie de réglage (3) est en forme de plaque.

7. Dispositif selon l'une des revendications 1 à 6, caractérisé en ce que l'appui nasal (4, 14) fait partie d'une pièce amovible (37) pouvant être fixée sur le boîtier (1) ou sur la partie de réglage (3).

8. Dispositif selon la revendication 7, caractérisé en ce qu'il est prévu une pluralité de pièces amovibles (37) avec des évidements (4, 14) pour différentes formes de nez.

**Claims**

1. Tool for measuring the distance of the eyes from the root of the nose with a nose overlay piece (4, 14), characterised in that the tool is designed asymmetrically and has a measuring mark (11) on only one side of the nose overlay piece (4, 14), said mark (11) being adjustable relative to the nose overlay piece (4, 14) by means of a slide (8) which is moved by an adjusting spindle (17), and in that a handle part (2) is provided on the other side of the nose overlay piece (4, 14), said handle part (2) having a viewing window (7) for a numerical measurement reading on each of its sides which in use face towards the operator.

2. Tool as claimed in claim 1, characterised in that the handle part (2) is provided with a closable opening to receive at least one electric battery.

3. Tool as claimed in claim 2, characterised in that a switch for the numerical measurement reading (digital reading) is a press-button switch, spring-loaded in the switch-off direction.

4. Tool as claimed in one of the claims 1 to 3, characterised in that the handle part (2) consists of two plastic half-shells (28, 29).

5. Tool as claimed in one of the claims 1 to 4, characterised in that a transparent measuring disc (10) disposed in the adjusting part (3) is arranged near to the nose overlay piece (4, 14).

6. Tool as claimed in one of the claims 2 to 5, characterised in that the slide (8) mounted in the adjusting part (3) is designed plate-shaped.

7. Tool as claimed in one of the claims 1 to 6, characterised in that the nose overlay piece (4, 14) is a component part of an interchangeable part (37) which can be fitted on the housing (1) or on the adjusting part (3).

8. Tool as claimed in claim 7, characterised in that several interchangeable parts (37) are provided with different recesses (4, 14) for the nose.

FIG. 2

18  3  4  28  2

36  20  19  10  IV  III  12  26  23  29  1  9  21  22

3  1  2  7  22

FIG. 1

20  10  11  5  4  6  25  23  26  27  24  II

0 135 598

5

28  22

FIG. 3

36  20  17  8  31  12  30  2  29

FIG. 4

FIG. 5

VIII →

FIG. 6

FIG. 7

FIG. 8

0 135 598

FIG. 9

0 135 598

FIG. 11

FIG. 10